# NEUE EUROPÄISCHE PATENTSCHRIFT

(11) **EP 1 479 353 B2**
(45) Veröffentlichungstag und Bekanntmachung des Hinweises auf die Entscheidung über den Einspruch: **08.10.2014**
(45) Hinweis auf die Patenterteilung: 28.03.2007
(21) Anmeldenummer: 03011679.2
(22) Anmeldetag: 23.05.2003
(51) Int. Cl.: A61B 18/12

(54) **Steuereinrichtung**
Control panel
Dispositif de commande

(43) Veröffentlichungstag der Anmeldung: 24.11.2004
(73) Patentinhaber: KLS Martin GmbH + Co. KG, 79224 Umkirch (DE)
(72) Erfinder: Hölscher Prof. Dr., Uvo M., 48565 Steinfurt (DE); Uphoff Dr.-Ing, Peter, 79211 Denzlingen (DE); Hug Dr.-Ing, Bernhard, 79111 Freiburg (DE); Frankus, Frank, 79111 Freiburg (DE)
(74) Vertreter: Kohler Schmid Möbus

(56) Entgegenhaltungen:
- WO-A1-00/73867
- DE-A- 3 923 024
- DE-A- 19 943 792
- DE-A1- 10 022 588
- DE-A1- 19 951 100
- US-A- 4 739 759
- US-A- 5 409 485
- US-A1- 2002 115 917

## Beschreibung

Die Erfindung betrifft eine Steuereinrichtung einer Steuereinrichtung für eine Vorrichtung der Medizintechnik, wobei die Steuereinrichtung mehrere Betriebsparameteranzeigefelder, welche einstellbare Parameter eines Betriebszustandes der Vorrichtung anzeigen, und ein Bedienelement zum Einstellen der Parameter aufweist, wobei die Betriebsparameteranzeigefelder jeweils in einer der Betriebsart und/oder dem Betriebszustand zugeordneten Farbe farbig markiert oder unterlegt sind.

Eine Steuereinrichtung der eingangs beschriebenen Art ist beispielsweise durch die DE 3923024 bekannt geworden.

Mithilfe der bekannten Steuereinrichtung sind Steuerbefehle und Parameter für ein vorgesehenes Arbeitsprogramm wählbar.

Jeder Einstellvorgang ist dadurch charakterisiert, dass der Blick des Anwenders erst ein bestimmtes Betriebsparameteranzeigefeld abliest, er danach mit dem Auge die Stelle für das Bedienelement sucht, um nach ihm greifen zu können, danach wieder auf das Betriebsparameteranzeigefeld sieht und ohne das Bedienelement anzuschauen so lange verstellt, bis der gewünschte Einstellwert in der Anzeige erscheint. Der Einstellvorgang ist also mit mehrmaligen Blickwechseln verbunden. Insbesondere in Situationen, in denen viele Parameter in gedrängter Form auf dem Betriebsparameteranzeigefeld erscheinen, liegt eine Fehlermöglichkeit in der Verwechselung des abgelesenenen Parameters. Es ist Ziel der Erfindung, diese Fehlermöglichkeit durch eine bessere Benutzerführung und eindeutige Markierungen zu verringern und damit die Sicherheit zu erhöhen.

Die Erfindung ist insbesondere auf dem Gebiet der Elektrochirurgie anwendbar.

Die bekannte mit Hochfrequenz arbeitende Elektrochirurgie betrifft das Schneiden oder Koagulieren von menschlichem Gewebe mithilfe elektrischer Energie. Durch die Verwendung geeigneter Elektroden kann das Gewebe thermisch aufgetrennt, und die angeschnittenen Gefäße können koaguliert werden. Dadurch wird ein Bluten im Schneidebereich weitgehend unterbunden.

Die Steuereinrichtung, d.h. der Hochfrequenzgenerator für die elektrochirurgische Anwendung, und der Operationstisch sind in der Regel lokal getrennt angeordnet. Die Steuereinrichtung muss nicht zwingend vom Chirurgen bedient werden und kann multifunktional verwendet werden, d.h. zum Schneiden und zum Koagulieren unterschiedlicher menschlicher Organe.

Wenn nur ein zentrales Bedienelement ausgebildet ist, muss vom Chirurgen bzw. vom Assistenten eine erhöhte Aufmerksamkeit aufgewendet werden, damit bei der Operation sichergestellt ist, dass die richtige Funktion mit den richtigen Betriebsparametern angesteuert wird. Ist nur ein zentrales Bedienelement zur Verstellung verschiedener Parameter vorhanden, muss es vor der Verstellung einem der Parameter zugeordnet werden. Häufig wird nach einem Verstellvorgang diese Zuordnung wieder aufgehoben, um eine versehentliche Verstellung auszuschließen. Die Funktion des Bedienelementes kann also verschiedene Zustände annehmen:
- inaktiv
- zugeordnet dem Parameter 1
- zugeordnet dem Parameter 2
- zugeordnet dem Parameter ...

Dem Bedienelement ist primär sein Aktivierungszustand und seine Betriebsart oder Betriebsmodus nicht ansehbar.

Der Anmelder hat sich die Aufgabe gestellt, einen Beitrag zur Sicherheitserhöhung beim Einsatz derartiger Steuereinrichtungen zu leisten.

Die Lösung der Aufgabe besteht nun gemäß Anspruch 1 darin, dass ein einziges zentrales Bedienelement vorgesehen ist und dass im Bereich des Bedienelements zusätzliche Mittel zur Darstellung der dieser Betriebsart und/oder diesem Betriebszustand zugeordneten Farbe vorgesehen sind. Die aktuell eingestellte Betriebsart und/oder der aktuell eingestellte Betriebszustand werden in ergonomisch sinnvoller Weise direkt am Bedienelement visuell angezeigt oder das Bedienelement ändert seine Farbe entsprechend. Es findet mittels geeigneter elektrischer und/oder elektronischer Schaltungen eine automatische Verknüpfung der farbigen Markierung der Betriebsparameteranzeigefelder und der farbigen Markierung des Bedienelements statt, so dass an beiden Stellen dieselbe farbige Markierung für die Betriebsart und/oder den aktivierten Betriebszustand gegeben ist. Wenn der Anwender am Bedienelement Betriebsparameter für eine Betriebsart und/oder einen bestimmten Betriebszustand verändert, ist durch die farbige Markierung des Bedienelements auch die Betriebsart und/oder der Betriebszustand angezeigt. Dies erhöht die Sicherheit. Ist das Bedienelement nicht aktiviert, wird das Bedienelement nicht farbig markiert und erscheint in einer neutralen Grundfarbe.

Einfache technische Realisierungen der Erfindung können darin bestehen, dass das Bedienelement durch einen Drehknopf und dass die Mittel zur Darstellung der Betriebsart und/oder des Betriebszustandes durch einen Leuchtring ausgebildet sind. Eine andere Ausbildung ist durch farbige Hinter-/Beleuchtung des Drehknopfes gegeben. Eine leichte Handhabung bei der Einstellung der Betriebsart und/oder des Betriebszustands wird mit einer leichten Erkennbarkeit der Betriebsart und/oder des Betriebszustandes unterstützt. Mehrere farbige Leuchtmittel, z.B. Leuchtdioden, stellen sicher, dass die jeweilige einer Betriebsart und/oder einem Betriebszustand zugeordnete Farbe am Drehknopf auf einfache Weise ausreichend wahrnehmbar dargestellt werden kann.

Die Erfindung kann sinnvoll auf dem Gebiet der Elektrochirurgie genutzt werden. Ähnliche Anwendungen bei anderen Steuerungen sind möglich.

Ein bevorzugtes Ausführungsbeispiel der Erfindung ist in der Zeichnung schematisch dargestellt und wird nachfolgend mit Bezug zu den Figuren der Zeichnung näher erläutert. Es zeigt:
- **Fig. 1**: eine Frontansicht einer Steuereinrichtung;
- **Fig. 2**: den näheren Aufbau eines Leuchtrings an einem Bedienelement der Steuereinrichtung nach Fig. 1;

Die Erfindung wird beispielhaft anhand eines HF-Chirurgiegenerators zum elektrochirurgischen Schneiden und Koagulieren von Gewebe erklärt. Es ist aber auch denkbar, die Erfindung in Verbindung mit beliebigen anderen Steuereinrichtungen innerhalb oder außerhalb der Medizintechnik einzusetzen.

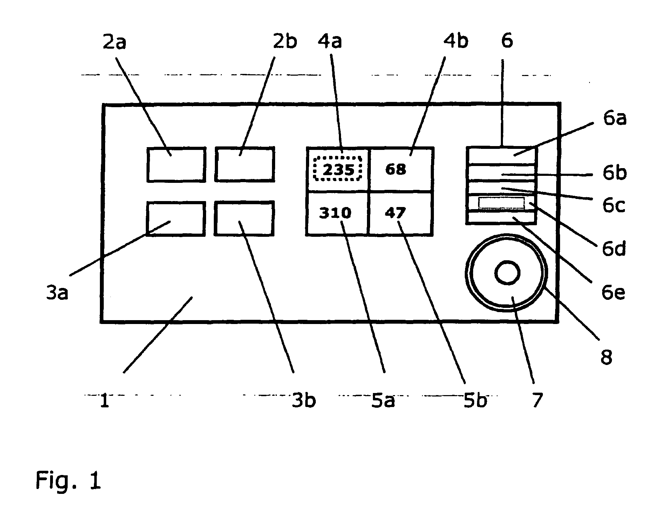

Aus der **Fig. 1** ist der wesentliche Aufbau eines Bedienfeldes **1** einer Steuereinrichtung zum Schneiden und Koagulieren von Gewebe in der Elektrochirurgie ersichtlich. Das Bedienfeld 1 umfasst neben in der Fig. 1 nicht gezeigten Anschlüssen und ggf. weiteren Elementen Betätigungstasten **2a**, **2b** sowie **3a** und **3b**, Betriebsparameteranzeigefelder **4a** und **4b** sowie **5a** und **5b**, eine Programmartenübersicht **6** mit Programmanzeigefeldern **6a** bis **6e** und ein Bedienelement in Gestalt eines Drehknopfes **7** mit Leuchtring **8**.

Es sind folgende Zuordnungen eingerichtet:
Betätigungstaste 2a zu Betriebsparameteranzeigefeld 4a;
Betätigungstaste 2b zu Betriebsparameteranzeigefeld 4b;
Betätigungstaste 3a zu Betriebsparameteranzeigefeld 5a;
Betätigungstaste 3b zu Betriebsparameteranzeigefeld 5b.

Eine Betätigung der zugeordneten Betätigungstaste aktiviert die jeweilige Betriebsparameteranzeige und gibt die Möglichkeit der Einstellung frei.

Der Betriebsparameter des Feldes 4a betrifft das elektrochirurgische Schneiden unter Flüssigkeit. Der Betriebsparameter des Feldes 5a betrifft das elektrochirurgische Schneiden unter Gas. Der Betriebsparameter des Feldes 4b betrifft das elektrochirurgische Koagulieren unter Flüssigkeit. Der Betriebsparameter des Feldes 5b betrifft das elektrochirurgische Koagulieren unter Gas.

Wenn nun mithilfe des aktivierten Programmanzeigefeldes 6d - in der Figur 1 bedeutet gepunktet dargestellt = aktiviert - ein bestimmtes Programm zur Behandlung eines Körperbereichs wie des Thorax ausgewählt ist, können in einem nächsten Schritt die erforderlichen Betriebsparameter eingestellt werden. Hierzu ist vorgesehen, dass über die Betätigungstaste 2a ein Betriebsparameteranzeigefeld, im vorliegenden Fall das Feld 4a, angewählt ist. Der einzustellende Wert, hier die Leistung von 235 Watt, wird numerisch angezeigt. Neben einer bestimmten Voreinstellung dieses Betriebsparameters für das Schneiden ist der Wert durch die Betätigung des zentralen Drehknopfes 7 veränderbar. Zum besseren Verständnis ist anzumerken, dass sich die Behandlungen Schneiden und Koagulieren vor allem durch die Leistung unterscheiden.

Daher ist es erforderlich, dass der Anwender jederzeit die gewählten bzw. die einzustellenden Betriebsparameter sofort erkennt, wenn der zentrale Drehknopf 7 betätigt wird. Hierzu ist es erfindungsgemäß vorgesehen, dass dem Schneiden eine erste Farbe, beispielsweise Gelb, und dem Koagulieren eine zweite Farbe, beispielsweise Blau, zugeordnet ist. Aus diesen Gründen sind die Betätigungstasten 2a, 3a und die Betriebsparameteranzeigefelder 4a, 5a in der ersten Farbe (Gelb) und die Betätigungstasten 2b, 3b und die Betriebsparameteranzeigefelder 4b, 5b in der zweiten Farbe (Blau) unterlegt. Weiterhin ist der zentrale Drehknopf 7 von einem Leuchtring 8 umgeben, so dass der Drehknopf 7 in Abstimmung zu den Betriebsparameteranzeigefeldern 4a, 4b, 5a, 5b von der Farbe umleuchtet wird, die der Unterlegung des jeweiligen Betriebsparameteranzeigefeldes entspricht. Der Anwender kann anhand seiner Information über die Zuordnung der Farben (Schneiden = Gelb; Koagulieren = Blau) und der Umleuchtung des Drehknopfes 7 sofort erkennen, dass er den Betriebsparameter für das Schneiden oder das Koagulieren ändert. Die zur Verstellung bereite, momentan aktive Betriebszustand der zu bedienenden Steuereinrichtung ist am Drehknopf 7 somit deutlich hervorgehoben.

Wenn am Menüfeld 6 eine allgemeine Einstellung ausgewählt ist, welche keine Primärfunktion (Schneiden oder Koagulieren) direkt betrifft, ist der Drehknopf 7 weiß umleuchtet.

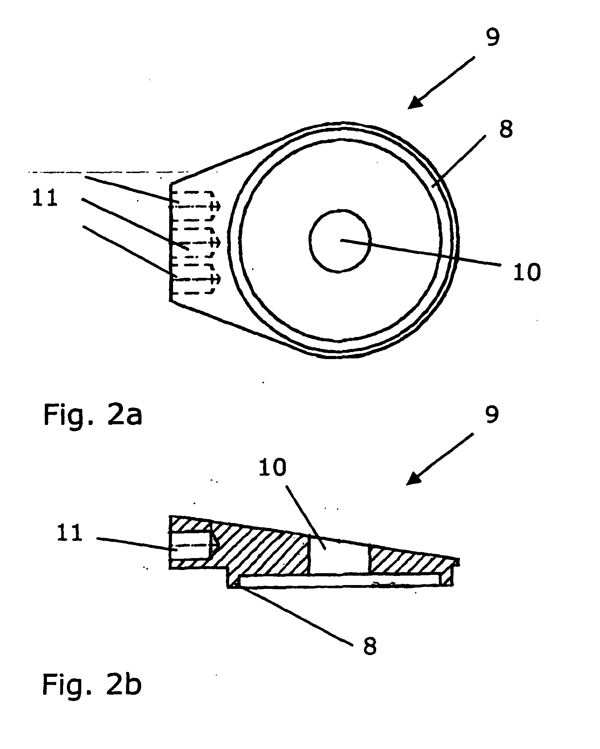

In einer Draufsicht und einer Schnittdarstellung einer lichtdurchlässigen Halterung **9** mit einer Drehachse **10** für den in der Fig. 2 nicht dargestellten Drehknopf 7 ist gemäß **Fign. 2a und 2b** zu erkennen, dass drei Aufnahmen **11** für Leuchtdioden ausgebildet sind, welche unterschiedliches farbiges Licht erzeugen. Dieses Licht wird in die Halterung 9 eingestrahlt, so dass der Leuchtring 8 zur Aufnahme des Drehknopfes 7 angestrahlt wird und den Drehknopf 7 leuchtend umgibt.

### BEZUGSZEICHENLISTE

- 1: Bedienfeld
- 2a: Betätigungstaste
- 2b: Betätigungstaste
- 3a: Betätigungstaste
- 3b: Betätigungstaste
- 4a: Betriebsparameteranzeigefeld
- 4b: Betriebsparameteranzeigefeld
- 5a: Betriebsparameteranzeigefeld
- 5b: Betriebsparameteranzeigefeld
- 6: Programmartenübersicht
- 7: Drehknopf
- 8: Leuchtring
- 9: Halterung
- 10: Drehachse
- 11: Aufnahme

## Patentansprüche

1. Steuereinrichtung für eine Vorrichtung der Medizintechnik, wobei die Steuereinrichtung mehrere Betriebsparameteranzeigefelder(4a, 4b, 5a, 5b), welche einstellbare Parameter eines Betriebszustandes der Vorrichtung anzeigen, und ein Bedienelement (7) zum Einstellen der Parameter aufweist, wobei die Betriebsparameteranzeigefelder (4a, 4b, 5a, 5b) jeweils in einer der Betriebsart und/oder dem Betriebszustand zugeordneten Farbe farbig markiert oder unterlegt sind, **dadurch gekennzeichnet, dass** ein einziges zentrales Bedienelement (7) vorgesehen ist und dass im Bereich des Bedienelements (7) zusätzliche Mittel(8) zur Darstellung der dieser Betriebsart und/oder diesem Betriebszustand zugeordneten Farbe vorgesehen sind.

2. Steuereinrichtung nach Anspruch 1, **dadurch gekennzeichnet, dass** das Bedienelement durch einen Drehknopf (7) und dass die Mittel zur Darstellung der Betriebsart und/oder des Betriebszustandes durch einen Leuchtring (8) ausgebildet sind.

3. Steuereinrichtung nach Anspruch 1, **dadurch** gekenntzeichnet, **dass** die farbige Markierung des Bedienelementes (7) auch anzeigt, ob sich das Bedienelement (7) zur Verstellung einer Betriebsart und/oder eines Betriebszustandes in einem aktivierten Zustand befindet oder ob das Bedienelement (7) inaktiv ist.

4. Steuereinrichtung nach Anspruch 2, **dadurch gekennzeichnet, dass** die Beleuchtung des Leuchtrings (8) durch mehrere farbige Leuchtdioden ausgebildet ist.

5. Steuereinrichtung nach einem oder mehreren der vorhergehenden Ansprüche, **dadurch gekennzeichnet, dass** die Steuereinrichtung zur Durchführung des elektrochirurgischen Schneidens und Koagulierens ausgebildet ist.

6. Steuereinrichtung nach Anspruch 4, **dadurch gekennzeichnet, dass** eine erste Farbe der Betriebsart Schneiden, eine zweite Farbe der Betriebsart Koagulieren zugeordnet ist.

7. Steuereinrichtung nach Anspruch 6, **dadurch gekennzeichnet, dass** eine dritte Farbe dem Betriebszustand "Allgemeine Einstellungen" zugeordnet ist und dass keine farbige Beleuchtung bzw. die Materialgrundfarbe einen für die Verstellbarkeit inaktiven Betriebszustand kennzeichnet.

## Claims

1. Control means for a mechanical engineering device, wherein the control means comprises several operational parameter display fields (4a, 4b, 5a, 5b) which display adjustable parameters of an operational state of the device, and a control element (7) for setting the parameters, wherein the operational parameter display fields (4a, 4b, 5a, 5b) are each marked or highlighted with a color which is associated with the operational mode and/or operational state, **characterized in that** one single central control element (7) is provided, and additional means (8) for displaying the color associated with this operational mode and/or this operational state are provided in the region of the control element (7).

2. Control means according to claim 1, **characterized in that** the control element is formed by a turning knob (7) and the means for displaying the operational mode and/or the operational state are formed by an illuminated ring (8).

3. Control means according to claim 1, **characterized in that** the colored marking of the control element (7) also shows whether the control element (7) is in an activated state to adjust an operational mode and/or an operational state, or whether the control element (7) is inactive.

4. Control means according to claim 2, **characterized in that** illumination of the illuminated ring (8) is provided by several colored light emitting diodes.

5. Control means according to any one or more of the preceding claims, **characterized in that** the control means is designed to perform electrosurgical cutting and coagulation.

6. Control means according to claim 4, **characterized in that** a first color is associated with the operational mode of cutting, and a second color is associated with the operational mode of coagulation.

7. Control means according to claim 6, **characterized in that** a third color is associated with the operational state "general settings" and that colorless illumination or the basic color of the material characterizes an operational state in which adjustment is inactive.

## Revendications

1. Dispositif de commande destiné à un dispositif de technique médicale, le dispositif de commande présentant plusieurs zones d'affichage des paramètres de fonctionnement (4a, 4b, 5a, 5b) qui indiquent des paramètres réglables relatifs à un état de fonctionnement du dispositif, et un élément de réglage (7) servant à régler les paramètres, les zones d'affichage des paramètres de fonctionnement (4a, 4b, 5a, 5b) étant marquées ou insérées respectivement dans une couleur attribuée au mode de fonctionnement et/ou à l'état de fonctionnement, **caractérisé en ce qu'**un seul élément de réglage central (7) est prévu et **en ce que** dans la zone de l'élément de réglage (7), des moyens additionnel (8) de représentation de la couleur attribuée à ce mode de fonctionnement et/ou à cet état de fonctionnement sont prévus.

2. Dispositif de commande selon la revendication 1, **caractérisé en ce que** l'élément de réglage est formé par un bouton tournant (7) et **en ce que** les moyens de représentation du mode de fonctionnement et/ou de l'état de fonctionnement sont formés par un anneau lumineux (8).

3. Dispositif de commande selon la revendication 1, **caractérisé en ce que** le marquage de couleur de l'élément de réglage (7) indique également si l'élément de réglage (7) servant à régler un mode de fonctionnement et/ou un état de fonctionnement se trouve en un état activé ou si l'élément de réglage (7) est inactif.

4. Dispositif de commande selon la revendication 2, **caractérisé en ce que** l'éclairage de l'anneau lumineux (8) est formé par plusieurs diodes électroluminescentes de couleur.

5. Dispositif de commande selon l'une quelconque ou plusieurs revendications précédentes, **caractérisé en ce que** le dispositif de commande est conçu dans le but d'exécuter l'incision et la coagulation électrochirurgicales.

6. Dispositif de commande selon la revendication 4, **caractérisé en ce qu'**une première couleur est attribuée au mode de fonctionnement "Incision", une deuxième au mode de fonctionnement "Coagulation".

7. Dispositif de commande selon la revendication 6, **caractérisé en ce qu'**une troisième couleur est attribuée à l'état de fonctionnement "Réglages généraux" et **en ce qu'**aucun éclairage de couleur ou la couleur de base de matériau caractérise un état de fonctionnement inactif pour le réglage.
